(19) Europäisches Patentamt
European Patent Office
Office européen des brevets

(11) EP 1 264 841 A1

(12) **EUROPEAN PATENT APPLICATION**

(43) Date of publication:
**11.12.2002 Bulletin 2002/50**

(21) Application number: **01610061.2**

(22) Date of filing: **08.06.2001**

(51) Int Cl.[7]: **C07K 14/47**, C12N 15/12, C12N 5/10, C12Q 1/68, G01N 33/53

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **NOVO NORDISK A/S**
**2880 Bagsvaerd (DK)**

(72) Inventors:
- **Andersen, Gitte**
  **1800 Frederiksberg C (DK)**
- **Ek, Jakob**
  **2100 Kobenhavn (DK)**
- **Hansen, Torben**
  **2900 Hellerup (DK)**
- **Pedersen, Oluf Borbye**
  **2800 Lyngby (DK)**

# (54) DNA encoding a mutant peroxisome proliferator-activated receptor gamma coactivator-1 (PGC-1), detection methods and test kits therefor

(57) The present invention provides an isolated polynucleotide molecule comprising a nucleotide sequence encoding PGC-1, said nucleotide sequence containing a mutation associated with type 2 diabetes of at least one nucleotide, or comprising a fragment of the nucleotide sequence including said mutation.

EP 1 264 841 A1

## Description

### FIELD OF THE INVENTION

The present invention relates to a mutant DNA sequence encoding peroxisome proliferator-activated receptor-γ coactivator-1, a method of detecting a mutation in the gene encoding peroxisome proliferator-activated receptor-γ coactivator-1, as well as a diagnostic composition and a test kit for use in the method.

### BACKGROUND OF THE INVENTION

Type 2 diabetes, also known as non-insulin dependent diabetes mellitus (NIDDM), is the most common of all metabolic disorders and poses a major health problem worldwide. Type 2 diabetes results from defects in both insulin secretion and insulin action, but the exact underlying mechanism(s) causing the disease are not known. An elevation of hepatic glucose production contributes significantly to causing fasting hyperglycemia, whereas decreased insulin-mediated glucose uptake by muscle and fat is a major contributor to postprandial hyperglycemia. Moreover, the metabolic fate of glucose taken up by muscle is not normal in people with type 2 diabetes. For example muscle glycogen synthase activity and glycogen synthesis have been shown to be impaired in type 2 diabetes. The available treatments do not allow for a complete normalisation of the metabolic state and some of them are associated with side effects. The metabolic derangements created by hyperglycemia, together with the strong association between type 2 diabetes, obesity, hypertension, and hyperlipidemia, lead to an extensive list of long-term complications, including a high rate of cardiovascular death due to accelerated atherosclerosis, as well as typical complications of diabetes such as retinopathy, nephropathy, and neuropathy.

There is extensive circumstantial evidence from family investigations including studies in twins and from studies of hybrid populations descended from high- and low-risk ancestral populations in favour of genetic determinants for the common late onset form of type 2 diabetes. It is also likely that type 2 diabetes in many cases is polygenic and it is suggested that subsets of patients display changes in various diabetes susceptibility genes thereby adding to the heterogeneity of type 2 diabetes.

As the symptoms of type 2 diabetes usually occur up to years after the onset of the disease and as type 2 diabetes is often first diagnosed when the long-term complications appear, there is a strong need for methods which enable an earlier diagnosis of type 2 diabetes. One such method could involve the detection of such genetic determinants associated with susceptibility for developing type 2 diabetes.

### SUMMARY OF THE INVENTION

According to the present invention, it has now been found that variability in the *PGC-1* (GenBank Accession Number AF106698) gene (SEQ ID NO: 1) confers susceptibility to type 2 diabetes and that a widespread missense polymorphism of this gene is reproducibly associated with type 2 diabetes. It is at present assumed that one or more of the mutations may be involved in or associated with the etiology of type 2 diabetes, and their presence may therefore be diagnostic for type 2 diabetes and possibly also other disorders associated with type 2 diabetes, like obesity, hyperlipidemia and hypertension. The variability of the gene may be used as a diagnostic tool to identify subjects who are at an increased risk of developing type 2 diabetes. The variant may also identify subjects with variable response to drugs which act via the peroxisome proliferator-activated receptor-γ, in other words the variant might be useful for tailoring of antidiabetic medication. The variant may also point to a new gene which could be of importance for development of new drugs.

Accordingly, the present invention encompasses an isolated polynucleotide molecule comprising a nucleotide sequence encoding PGC-1, said nucleotide sequence containing a mutation associated with type 2 diabetes of at least one nucleotide, or comprising a fragment of the nucleotide sequence including said mutation.

The present invention also encompasses a recombinant vector, especially an expression vector, comprising a polynucleotide according to the present invention.

The present invention also encompasses a cell line or a transgenic non-human mammal containing a polynucleotide or a recombinant vector according to the present invention.

The present invention also encompasses a method of detecting the presence of a mutation in the gene encoding PGC-1, the method comprising obtaining a biological sample from a subject and analysing the sample for a mutation associated with type 2 diabetes of at least one nucleotide in the *PGC-1* sequence.

The present invention also encompasses a diagnostic composition for determining predisposition to type 2 diabetes, or conditions often associated with type 2 diabetes, in a subject, the composition comprising a polynucleotide according to the present invention.

The present invention also encompasses a test kit for detecting the presence of a mutation associated with type 2 diabetes in the gene encoding PGC-1, the kit comprising a first polynucleotide comprising a nucleotide sequence corresponding to at least part of the gene encoding PGC-1 and containing a mutation of at least one nucleotide, which mutation corresponds to the mutation the presence of which in the gene encoding PGC-1 is to be detected and optionally a second polynucieotide comprising a nucleotide sequence corresponding to at least part of the wild-type gene encoding PGC-1.

The present invention also encompasses a test kit for detecting the presence of a mutation associated with type 2 diabetes in the gene encoding PGC-1 comprising means for amplifying DNA, and a labelled polynucleotide comprising a nucleotide sequence corresponding to at least part of the gene encoding PGC-1 and containing a mutation of at least one nucleotide, which mutation corresponds to the mutation the presence of which in the gene encoding PGC-1 is to be detected.

Further embodiments will become apparent from the following detailed description.

## FIGURES

Figure 1 shows a schematic presentation of identified *PGC-1* variants and approximate positions of identified variants relative to known functional domains. LXXLL, recognition site (LXXLL motif); PKAP, protein kinase A phosphorylation consensus site; SRD, serine and arginine rich domain; RRM, RNA recognition motif (Esterbauer et al (1999), Genomics 62, 98-102).

## DEFINITIONS

"Corresponding to", when used in reference to a nucleotide or amino acid sequence, indicate the position in the second sequence that aligns with the reference position when two sequences are optimally aligned.

The term "isolated", when applied to a polynucleotide, denotes that the polynucleotide has been removed from its natural genetic milieu. Such isolated molecules are those that are separated from the natural environment and include cDNA and genomic clones.

A "polynucleotide" is a single- or doublestranded polymer of nucleotides such as deoxyribonucleotide or ribonucleotide bases linked together by phosphodiester (5'-3') bonds and read from the 5' to the 3' end. Polynucleotides include RNA and DNA, and may be isolated from natural sources, synthesized *in vitro,* or prepared form a combination of natural and synthetic molecules. It will be recognized by those skilled in the art that the two strands of a double-stranded polynucleotide may differ slightly in length and that the ends thereof may be staggered as a result of enzymatic cleavage; thus all nucleotides within a double-stranded molecule may not be paired. As the skilled person will recognize, this definition of a polynucleotide does also comprise what is known as an oligonucleotide, that is a polynucleotide containing a small number of nucleotides.

A "DNA construct" is a polynucleotide as defined above as a single- or doublestranded polymer of deoxyribonucleotide bases.

## DETAILED DESCRIPTION OF THE INVENTION

The present invention provides an isolated polynucleotide molecule comprising a nucleotide sequence encoding PGC-1, said nucleotide sequence containing a mutation associated with type 2 diabetes of at least one nucleotide, or comprising a fragment of the nucleotide sequence including said mutation.

SEQ ID NO: 1 is the wild-type DNA sequence coding for PGC-1 (GenBank Accession Number AF106698) and SEQ ID NO: 2 is the wild-type amino acid sequence of PGC-1. Those skilled in the art will recognize that the DNA sequence in SEQ ID NO: 1 also provides the RNA sequence encoding SEQ ID NO: 2 by substituting U for T. Those skilled in the art will also readily recognise that, in view of the degeneracy of the genetic code, considerable sequence variation is possible among the polynucleotide molecules according to the present invention. Furthermore, the polynucleotide molecules may contain other sequence variations corresponding to amino acid substitutions in SEQ ID NO: 2 as long as this does not interfere with the utility of the polynucleotides according to the purpose of the present invention. Such sequence variations could for instance correspond to a genetic variation within a specific population, a member of which is being diagnosed for susceptibility for developing type 2 diabetes or other disorders associated with type 2 diabetes, like obesity, hyperlipidemia and hypertension, but could also be related to other amino acid substitutions of interest. Those skilled in the art will also recognize that the polynucleotides according to the present invention may also contain more than one mutation associated with type 2 diabetes or other disorders associated with type 2 diabetes.

The length of the polynucleotides according to the present invention may vary widely depending on the intended use. For use as a polynucleotide probe for hybridisation purposes, the polynucleotide may be as short as for instance 17 nucleotides or even shorter. For expression in a cell line or a transgenic non-human mammal as defined above, the polynucleotide according to the present invention will typically comprise the full-length DNA sequence encoding PGC-1.

The polynucleotide of the present invention comprising the mutation in the nucleotide sequence encoding PGC-1 may suitably be of genomic DNA or cDNA origin, for instance obtained by preparing a genomic or cDNA library and screening for DNA sequences coding for all or part of the PGC-1 by hybridisation using synthetic oligonucleotide probes in accordance with standard techniques (cf. Sambrook et al., Molecular Cloning: A Laboratory Manual, 2nd Ed., Cold Spring Harbor, 1989). The probes used should be specific for the mutation. Alternatively, the DNA molecule encoding wild-type PGC-1 may be modified by site-directed mutagenesis using synthetic oligonucleotides containing the mutation for homologous recombination in accordance with well-known procedures. The polynucleotides, especially the DNA constructs, according to the present invention, may also be prepared by polymerase chain reaction using specific primers, for instance as described in US 4,683,202, or Saiki et al. (1988), Science 239, 487-491.

The polynucleotides, especially the DNA constructs, of the present invention may also be prepared synthetically by established standard methods, e.g. the phosphoamidite method described by Beaucage and Caruthers (1981), Tetrahedron Letters 22, 1859-1869, or the method described by Matthes et al. (1984), EMBO Journal 3, 801-805. According to the phosphoamidite method, oligonucleotides are synthesized, e.g. in an automatic DNA synthesizer, purified, annealed and ligated. This procedure may preferably be used to prepare fragments of the PGC-1 encoding DNA sequence.

In one embodiment, the present invention provides a polynucleotide according to the present invention comprising a nucleotide sequence as shown in the Sequence Listing as SEQ ID NO: 1 containing a mutation associated with type 2 diabetes of at least one nucleotide or comprising a fragment of the nucleotide sequence shown in the Sequence Listing as SEQ ID NO: 1 including said mutation.

In a further embodiment, the present invention provides a polynucleotide according to the present invention, where said mutation gives rise to at least one amino acid substitution in PGC-1.

In a further embodiment, the present invention provides a polynucleotide according to the present invention, where said mutation gives rise to a substitution of Gly to Ser in the position corresponding to position 482 of the amino acid sequence shown in the Sequence Listing as SEQ ID NO: 2.

In a further embodiment, the present invention provides a polynucleotide according to the present invention, where said mutation corresponds to a mutation of G in position 1564 in SEQ ID NO: 1 to A.

In a further embodiment, the present invention provides a polynucleotide according to the present invention where said polynucleotide is a DNA construct.

The present invention provides a recombinant vector, especially an expression vector, comprising a polynucleotide according to the present invention.

The recombinant vector into which a polynucleotide according to the present invention is inserted may be any vector that conveniently may be subjected to recombinant DNA procedures. The choice of vector will often depend on the host cell into which it is to be introduced. Thus, the vector may be an autonomously replicating vector, i.e. a vector that exists as an extrachromosomal entity, the replication of which is independent of chromosomal replication, e.g. a plasmide. Alternatively, the vector may be one which, when introduced into a host cell, is integrated into the host cell genome and replicated together with the chromosome(s) into which it has been integrated (e.g. a viral vector).

In the vector, the mutant DNA sequence encoding PGC-1 may be operably connected to a suitable promoter sequence. The promoter may be any DNA sequence, which shows transcriptional activity in the host cell of choice and may be derived from genes encoding proteins either homologous or heterologous to the host cell. Examples of suitable promoters for directing the transcription of the mutant DNA encoding PGC-1 in mammalian cells are the SV40 promoter (Subramani et al. (1981), Mol. Cell Biol. 1, 854 -864), the MT-1 (metallothionein gene) promoter (Palmiter et al. (1983), Science 222, 809-814) or the adenovirus 2 major late promoter.

The mutant DNA sequence encoding PGC-1 may also be operably connected to a suitable terminator, such as the human growth hormone terminator (Palmiter et al., loc. cit.). The vector may further comprise elements such as polyadenylation signals (e.g. from SV40 or the adenovirus 5 Elb region), transcriptional enhancer sequences (e.g. the SV40 enhancer) and translational enhancer sequences (e.g. the ones encoding adenovirus VA RNAs).

The recombinant expression vector may further comprise a DNA sequence enabling the vector to replicate in the host cell in question. An example of such a sequence is the SV40 origin of replication. The vector may also comprise a selectable marker, e.g. a gene the product of which complements a defect in the host cell, such as the gene coding for dihydrofoiate reductase (DHFR) or one which confers resistance to a drug, e.g. neomycin, hygromycin or methotrexate.

The procedures used to ligate the DNA sequences coding for PGC-1, the promoter and the terminator, respectively, and to insert them into suitable vectors containing the information necessary for replication, are well known to persons skilled in the art (cf., for instance, Sambrook et al., loc.cit.).

The present invention provides a cell line or a transgenic non-human mammal containing a polynucleotide or a recombinant vector according to the present invention.

A cell line into which a polynucleotide or a recombinant vector according to the present invention may be introduced may be any cell in which the polynucleotide can be replicated, such as a prokaryotic cell, for example *Eschericia coli* or a eukaryotic cell, such as a vertebrate cell, e.g. a *Xenopus laevis* oocyte or a mammalian cell. The cell line into which a polynucleotide according to the present invention is introduced may also be a cell which is capable of producing PGC-1 and which has the appropriate signal transduction pathways. Such a cell is preferably a eukaryotic cell, such as a vertebrate cell, e.g. a *Xenopus laevis* oocyte or mammalian cell, in particular a mammalian cell. Examples of suitable mammalian cell lines are the COS (ATCC CRL 1650), BHK (ATCC CRL 1632, ATCC CCL 10), CHL (ATCC CCL39) or CHO (ATCC CCL 61) cell lines.

Methods of transfecting cells, such as prokaryotic cells and eukaryotic cells, such as mammalian cells, are described in e.g. Old RW, Primrose SB, Principles of gene manipulation - an introduction to genetic engineering. Fifth edition. Blackwell Science Ltd. Oxford, 1994.

Expressing DNA sequences introduced in such cells especially eukaryotic cells, and more especially mammalian cells, are described in e.g. Kaufman and Sharp (1982), J. Mol. Biol. 159, 601-621; Southern and Berg (1982), J. Mol. Appl. Genet. 1, 327-341; Loyter et al. (1982), Proc. Natl. Acad. Sci. USA 79, 422-426; Wigler et al. (1978), Cell 14, 725; Corsaro and Pearson (1981), Somatic Cell Genetics 7, 603; Graham and van der Eb (1973), Virology 52, 456; and Neumann et al. (1982), EMBO J. 1, 841-845.

A mutant DNA sequence encoding PGC-1 may then be expressed by culturing cells as described above in a suitable nutrient medium under conditions, which are conducive to the expression of the PGC-1-encoding DNA sequence. The medium used to culture the cells may be any conventional medium suitable for growing such a cell, such as medium suitable for growing mammalian cells, such as a serum-containing or serum-free medium containing appropriate supplements. Suitable media are available from commercial suppliers or may be prepared according to published recipes (e.g. in catalogues of the American Type Culture Collection).

A polynucleotide according to the present invention may also be introduced into a transgenic animal. A transgenic animal is one in whose genome a heterologous DNA sequence has been introduced. In particular, the transgenic animal is a transgenic non-human mammal, mammals being generally provided with appropriate signal transduction pathways. The mammal may conveniently be a rodent such as a rat or mouse. The mutant DNA sequence encoding PGC-1 may be introduced into the transgenic animal by any one of the methods previously described for this purpose. Briefly, the DNA sequence to be introduced may be injected into a fertilised ovum or cell of an embryo, which is subsequently implanted into a female mammal by standard methods, resulting in a transgenic mammal whose germ cells and/or somatic cells contain the mutant DNA sequence. For a more detailed description of a method of producing transgenic mammals, vide B. Hogan et al., Manipulating the Mouse Embryo, Cold Spring Harbor Laboratory, Cold Spring Harbor, New York. The mutant DNA sequence may also be introduced into the animal by transfection of fertilised ova with a retrovirus containing the DNA sequence, cf. R. Jaenisch (1976), Proc. Natl. Acad. Sci. USA 73, 1260-1264. A further method of preparing transgenic animals is described in Gordon and Ruddle (1983), Methods Enzymol. 101, 411-432.

The present invention also provides a method of detecting the presence of a mutation in the gene encoding PGC-1, the method comprising obtaining a biological sample from a subject and analysing the sample for a mutation associated with type 2 diabetes of at least one nucleotide in the *PGC-1* sequence.

In one embodiment a method according to the present invention comprises analysing said sample for a mutation associated with type 2 diabetes of at least one nucleotide in the *PGC-1* sequence, which mutation gives rise to at least one amino acid substitution in PGC-1.

In a further embodiment a method according to the present invention comprises analysing said sample for a mutation associated with type 2 diabetes of at least one nucleotide in the *PGC-1* sequence, which mutation gives rise to a substitution of Gly to Ser in the position corresponding to position 482 of the amino acid sequence shown in the Sequence Listing as SEQ ID NO: 2.

In a further embodiment a method according to the present invention comprises analysing said sample for a mutation associated with type 2 diabetes of at least one nucleotide in the *PGC-1* sequence, which mutation corresponds to a mutation of G in position 1564 in SEQ ID NO: 1 to A.

In a further embodiment of the present method of detecting the presence of a mutation in the *PGC-1* gene, a biological sample is obtained from a subject, DNA (in particular genomic DNA) is isolated from the sample and digested with a restriction endonuclease which cleaves DNA at the site of the mutation, and cleavage of the DNA within the gene encoding PGC-1 at this site is determined. After digestion, the resulting DNA fragments may be subjected to electrophoresis on an agarose gel. DNA from the gel may then be blotted onto a nitrocellulose filter and hybridised with a radiolabelled probe. The probe may conveniently contain a DNA fragment of the PGC-1 gene spanning the mutation (substantially according to the method of E.M. Southern (1975), J. Mol. Biol. 98, 503, e.g. as described by B. J. Conner et al. (1983), Proc. Natl. Acad. Sci. USA 80, 278-282).

In a variant of this embodiment, the DNA isolated from the sample may be amplified prior to digestion with the restriction endonuclease. Amplification may suitably be performed by polymerase chain reaction (PCR) using oligonucleotide primers based on the appropriate sequence of PGC-1 spanning the site(s) of mutation, essentially as described by Saiki et al. (1985), Science 230, 1350-1354. After amplification, the amplified DNA may be digested with the appropriate restriction endonudease and subjected to agarose gel electrophoresis. The restriction pattern obtained may be analysed, e.g. by staining with ethidium bromide and visualising bands in the gel by means of UV light. As a control, wild-type DNA encoding PGC-1 (i.e. not containing the mutation) may be subjected to the same procedure, and the restriction patterns may be compared.

In one embodiment of a method according to the present invention, a biological sample is obtained from a subject, DNA is isolated from the sample, the DNA is amplified and hybridised to a labelled polynucleotide comprising a nucleotide sequence encoding PGC-1, said nucleotide sequence containing a mutation associated with type 2 diabetes of at least one nucleotide, or comprising a fragment of the nucleotide sequence including said mutation, which mutation corresponds to the mutation the presence of which in the gene encoding PGC-1 is to be detected, and hybridisation of the labelled polynucleotide to the DNA is determined.

In a further embodiment of said method, the labelled polynucleotide comprises a nucleotide sequence as shown in the Sequence Listing as SEQ ID NO: 1 containing a mutation associated with type 2 diabetes of at least one nucleotide or comprising a fragment of the nucleotide sequence shown in the Sequence Listing as SEQ ID NO: 1 including said mutation.

In a further embodiment of said method, the labelled polynucleotide comprises a nucleotide sequence containing a mutation, which mutation gives rise to a substitution of Gly to Ser in the position corresponding to position 482 of the amino acid sequence shown in the Sequence Listing as SEQ ID NO: 2.

In a further embodiment of said method, the labelled polynucleotide comprises a nucleotide sequence containing a mutation corresponding to a mutation of G in position 1564 in SEQ ID NO: 1 to A.

In a further embodiment of said method, said labelled polynucleotide is a DNA construct.

In a further embodiment of said method the amplified DNA is hybridised to a second labelled polynucleotide comprising a DNA sequence corresponding to at least part of the wild-type gene encoding PGC-1, and hybridisation of said second labelled polynucleotide to the amplified DNA is determined.

In a further embodiment of said method, the label substance with which the labelled polynucleotide carrying the mutation is labelled is different from the label substance with which the second labelled polynucleotide corresponding to at least part of the wild-type DNA is labelled.

In a further embodiment the present invention provides a method according to the invention for determining predisposition to type 2 diabetes in a subject.

A further embodiment of a method according to the present invention is an adaptation of the method described by U. Landegren et al. (1988), Science 241, 1077-1080, which involves the ligation of adjacent oligonucleotides on a complementary target DNA molecule. Ligation will occur at the junction of the two oligonucleotides if the nucleotides are correctly base paired.

In a further embodiment of a method according to the present invention, the DNA isolated from the sample may be amplified using oligonucleotide primers corresponding to segments of the gene coding for PGC-1. The amplified DNA may then be analysed by hybridisation with a labelled polynucleotide comprising a DNA sequence corresponding to at least part of the gene encoding PGC-1 and containing a mutation of at least one nucleotide, which mutation corresponds to the mutation the presence of which in the gene encoding PGC-1 is to be detected. As a control, the amplified DNA may furthermore be hybridised with a further labelled polynucleotide comprising a DNA sequence corresponding to at least part of the wild-type gene encoding PGC-1. This procedure is, for instance, described by DiLella et al. (1988), Lancet 1, 497-499. Another PCR-based method which may be used in the present invention is the allele-specific PCR method described by R. Saiki et al. (1986), Nature 324, 163-166, or D.Y. Wu et al. (1989), Proc. Natl. Acad. Sci. USA 86, 2757-2760, which uses primers specific for the mutation in the *PGC-1* gene.

Other methods of detecting mutations in DNA are reviewed in U. Landegren (1992), GATA 9, 3-8. One of the currently preferred methods of detecting mutations is by single stranded conformation polymorphism (SSCP) analysis substantially as described by Orita et al. (1989), Proc. Natl. Acad. Sci. USA 86, 2766-2770, or Orita et al. (1989), Genomics 5, 874-879.

The label substance with which the polynucleotide may be labelled may be selected from the group consisting of enzymes, coloured or fluorescent substances, or radioactive isotopes.

Examples of enzymes useful as label substances are peroxidases (such as horseradish peroxidase), phosphatases (such as acid or alkaline phosphatase), β-galactosidase, urease, glucose oxidase, carbonic anhydrase, acetylcholinesterase, glucoamylase, lysozyme, malate dehydrogenase, glucose-6-phosphate dehydrogenase, β-glucosidase, proteases, pyruvate decarboxylase, esterases, luciferase, etc.

Enzymes are not in themselves detectable but must be combined with a substrate to catalyse a reaction the end product of which is detectable. Examples of substrates, which may be employed in the method according to the invention, include hydrogen peroxide/tetramethylbenzidine or chloronaphthole or o-phenylenediamine or 3-(p-hydroxyphenyl) propionic acid or luminol, indoxyl phosphate, p-nitrophenylphosphate, nitrophenyl galactose, 4-methyl umbelliferyl-D-galactopyranoside, or luciferin.

Alternatively, the label substance may comprise coloured or fluorescent substances, including gold particles, coloured or fluorescent latex particles, dye particles, fluorescein, phycoerythrin or phycocyanin.

In one embodiment, the labelled polynucleotide is labelled with a radioactive isotope. Radioactive isotopes, which may be used for the present purpose, may be selected from I-125, I-131, In-111, H-3, P-32, C-14 or S-35. The radioactivity emitted by these isotopes may be measured in a beta- or gamma-counter or by a scintillation camera in a manner known per se.

The present invention provides a diagnostic composition for determining predisposition to type 2 diabetes in a subject, the composition comprising a polynucleotide according to the present invention.

The present invention also provides a diagnostic composition for detecting the presence of a mutation in the gene encoding PGC-1, the composition comprising a polynucleotide according to the present invention.

The present invention also provides a test kit for detecting the presence of a mutation associated with type 2 diabetes in the gene encoding PGC-1, the kit comprising a first polynucleotide comprising a nucleotide sequence corresponding to at least part of the gene encoding PGC-1 and containing a mutation of at least one nucleotide, which mutation corresponds to the mutation the presence of which in the gene encoding PGC-1 is to be detected and optionally a second polynucleotide comprising a nucleotide sequence corresponding to at least part of the wild-type gene encoding PGC-1.

In one embodiment of the present invention, the first polynucleotide in said test kit is a polynucleotide according to the present invention.

In a further embodiment of the present invention, said test kit further comprises means for amplifying DNA.

The present invention also provides a test kit for detecting the presence of a mutation associated with type 2 diabetes in the gene encoding PGC-1, the kit comprising means for amplifying DNA, and a labelled polynucleotide comprising a nucleotide sequence corresponding to at least part of the gene encoding PGC-1 and containing a mutation of at least one nucleotide, which mutation corresponds to the mutation the presence of which in the gene encoding PGC-1 is to be detected.

In one embodiment of the present invention, the labelled polynucleotide in said test kit comprises a polynucleotide according to the present invention.

In a further embodiment of the present invention, said test kit further comprises a second labelled polynucleotide comprising a nucleotide sequence corresponding to at least part of the wild-type gene encoding PGC-1.

In a further embodiment of the present invention, the label substance with which the labelled polynucleotide in said kit carrying the mutation is labelled is different from the label substance with which the second labelled polynucleotide corresponding to at least part of the wild-type DNA is labelled.

In a further embodiment of the present invention, the second labelled polynucleotide in said test kit is a DNA construct.

In one embodiment, the present invention provides a test kit according to the present invention for determining predisposition to type 2 diabetes in a subject.

As elucidated in the following example, the *PGC-1* gene is associated with type 2 diabetes. Four gene variants of *PGC*-1 are tested, namely Ser74Leu, IVS2+52C→A, Gly482Ser, and Thr612Met were examined in an association study comprising 483 type 2 diabetic patients and 216 glucose tolerant control subjects.

Three of the variants (Ser74Leu, IVS2+52C→A and Thr612Met) showed no significant differences in allele frequencies between diabetic and control subjects. However, the Gly482Ser polymorphism, was more frequent among type 2 diabetic patients (37.0%) compared to glucose tolerant subjects (30.8%) ($p$=0.032) (Table 2). Testing this variant for association to type 2 diabetes in the second subject samples confirmed the association of the polymorphism to type 2 diabetes. Combining the initial and replication samples demonstrated a 1.34 fold increase in diabetes risk associated with the Ser-allele of Gly482Ser. Although this diabetes susceptibility effect appears to be small it translates into the considerable population attributable risk of 18% due to the high frequency of the risk allele.

*PGC-1* Gly482Ser variant is associated with type 2 diabetes in the examined populations and may be the causative polymorphism. Alternatively, the mutation may be a marker associated with another mutation in this or another gene, which other mutation is the one actually involved in disease etiology, for instance in linkage disequilibrium with a not yet identified aetiological variant. Gly482Ser is located in a part of the protein with unknown function and glycine at codon 482 is not conserved between man and mice. Clearly, functional studies are needed to elucidate whether the codon 482 variant itself has biological implications.

**Example 1**

Subjects and Methods

*Subjects*

Mutation analysis was performed in 53 type 2 diabetic patients (30 males, 23 females) recruited from the outpatient clinic at Steno Diabetes Center, Denmark. The age of the patients was 64 ± 9 years, age of diagnosis 57 ± 9 years, body mass index (BMI) 29.7 ± 4.9 kg/$m^2$, and $HbA_{1C}$ 8.3 ± 1.7% (mean ± S.D.). More than 70% of the patients fulfilled the WHO criteria for the metabolic syndrome, 31% of the patients were treated with diet alone, 65% with oral hypoglycaemic agents (OHA), and 4% with insulin alone or in combination with OHA.

The initial association studies were performed in a group of unrelated type 2 diabetic patients recruited from the outpatient clinic at Steno Diabetes Center during 1994-1997 and a group of unrelated glucose tolerant subjects without a known family history of diabetes randomly sampled during 1994-1997 from the Danish Central Population Register and all living in the same area of Copenhagen as the type 2 diabetic patients. In the group of type 2 diabetic patients (n = 483, 278 males, 205 females) the age was 61 ± 11 years, age of diagnosis 55 ± 11 years, BMI 29.0 ± 5.3 kg/$m^2$, and $HbA_{1C}$ 8.1 ± 1.6%. The patients were treated with diet alone (27%), with OHA (58%), or with insulin in combination with OHA (15%). In the group of glucose tolerant subjects (n = 216, 105 males, 111 females) the age was 52 ± 14 years, and BMI 25.3 ± 3.8 kg/$m^2$.

The association study used for replication comprised unrelated type 2 diabetic patients recruited from the outpatient clinic at Steno Diabetes Center during 1992-1993 and a population based sample of unrelated glucose tolerant subjects without a known family history of diabetes born in 1936 and examined during 1996-1997 at the Copenhagen County Centre of Preventive Medicine. In the group of type 2 diabetic patients (n = 201, 152 males, 49 females) the age was 55 ± 7 years, age of diagnosis 48 ± 8 years, BMI 29.8 ± 4.4 kg/$m^2$, and $HbA_{1C}$ 8.6 ± 1.7%. The patients were treated with diet alone (29%), with OHA (60%), or with insulin in combination with OHA (11%). In the group of glucose tolerant subjects (n = 293, 134 males, 159 females) the age was 60.5 ± 0.4 years and BMI 26.2 ± 3.7 kg/$m^2$.

Diabetes was diagnosed according to 1998 WHO criteria (Alberti KG, Zimmet PZ (1998) Definition, diagnosis and classification of diabetes mellitus and its complications. Part 1: diagnosis and classification of diabetes mellitus provisional report of a WHO consultation. Diabet Med 15, 539-553). All glucose tolerant subjects underwent a 75 g oral glucose tolerance test (OGTT). All participants were Danish Caucasians by self-identification. Informed written consent was obtained from all subjects prior to participation. The study was approved by the Ethical Committee of Copenhagen and was in accordance with the principles of the Declaration of Helsinki II.

*Biochemical assays*

Blood samples for measurement of serum levels of insulin, total cholesterol, high-density lipoprotein (HDL) cholesterol, triglycerides, and plasma glucose and free fatty acids (FFA) were drawn after a 12-hour overnight fast. Serum triglycerides, total serum cholesterol, serum HDL-cholesterol, serum low-density lipoprotein cholesterol and plasma FFA were analysed using enzymatic colorimetric methods (GPO-PAP and CHOD-PAP, Boehringer Mannheim, Germany and NEFA C, Wako, Germany). The plasma glucose concentration was analysed by a glucose oxidase method (Granutest, Merck, Darmstadt, Germany) and serum specific insulin (excluding des(31,32)- and intact proinsulin) was measured by ELISA (Dako insulin kit K6219, Dako Diagnostics Ltd. Ely, UK). $HbA_{1C}$ was measured by ion-exchange high performance liquid chromatography (non-diabetic reference range: 4.1 - 6.4%).

*Mutation analysis and genotyping*

The genetic analyses were performed on genomic DNA isolated from human leukocytes. The coding region of the *PGC-1* gene (EMBL #AF106698) including intron-exon boundaries (in total 3357 bp) was divided into 17 segments (sized 145-273 nucleotides) for SSCP and heteroduplex analysis. In the inventors laboratory this methodology has a sensitivity of more than 95% for detecting a variety of known mutations. The segments also included the 5' untranslated sequence of 90 bp. Primer sequences are listed in Table 1. PCR amplification was carried out in a volume of 25 μl containing 100 ng genomic DNA, 1×PCR-buffer, 0.2 μM of each primer, 0.2 mM dNTP, 10 mCi/ml $\alpha$-$^{32}$P-dCTP, 0.6 units AmpliTaq Gold polymerase (Perkin Elmer, Ca, USA) and $MgCl_2$ concentration as shown in Table 1. The cycling program was a denaturation step at 95°C for 15 min followed by 40 cycles of 94°C for 30 seconds, annealing at $T_{anneal}$ for 30 seconds, and elongation at 72°C for 60 seconds with a final elongation step at 72°C for 9 min using a GeneAmp 9600 thermal cycler (Perkin Elmer). The annealing temperatures are listed in Table 1. SSCP was performed at two different experimental settings as reported in Hansen T et al. (1997), Diabetes 46, 494-501 and aberrantly migrating samples were sequenced using fluorescent chemistry (Dye Primer Cycle Sequencing Ready Reaction Kit, Applied Biosystems, Ca, USA). The Ser74Leu and IVS2+52C→A variants were genotyped by PCR with primers PC2F-PC2RNY followed by digestion with *Dra*I and *Apa*I, respectively. The Gly482Ser variant was amplified with primers PC15F-PC17R and digested with *Hpa*II. The Thr612Met variant was amplified with primers PC8F-PC8R and digested with *Nla*III. All restriction enzyme digests were separated on 4% agarose gels.

*Statistical analysis*

Fisher's exact test was applied to examine for differences in allele frequencies between diabetic and non-diabetic subjects. A *p*-value of less than 0.05 was considered significant. All analyses were performed using Statistical Package for Social Science (SPSS) version 10.0. The genotype relative risk (GRR) was estimated by logistic regression from the genotype data using a log-additive model for the risk. Test for additivity gave a likelihood ratio statistic of 0.285 on 1 df ($p$ = 0.593). Population attributable risk was calculated in standard fashion as

$$(P_{He}\cdot(GRR-1)+P_{Ho}\cdot(GRR^2-1))/(1+P_{He}\cdot(GRR-1)+P_{Ho}\cdot(GRR^2-1)),$$

where P is the frequency of the risk genotype.

Results

The mutation screening covered the coding region of *PGC-1.* In the 53 diabetic patients a total of six different variants was identified (Figure 1): Ser74Leu (identified in 2 out of 53 patients), IVS2+52C→A (19 patients), Asp475Asp (13 patients), Gly482Ser (24 patients), Thr528Thr (37 patients), and Thr612Met (3 patients). The three variants, which predicted changes of amino acids and the prevalent intronic variant, IVS2+52C→A, were further examined in an association study comprising 483 type 2 diabetic patients and 216 glucose tolerant control subjects. All variants were in Hardy-Weinberg equilibrium. The allele frequencies of the Ser74Leu, IVS2+52C→A, and Thr612Met variants did not differ significantly between diabetic and non-diabetic subjects (Table 2).

The allele frequency of the Gly482Ser variant was higher among type 2 diabetic patients compared to glucose tolerant subjects (37.0% vs. 30.8%, $p$ = 0.032) (Table 2). In a replication study the differences in allele frequencies remained significant (38.1% vs. 30.4%, $p$ = 0.0135). The combined study yielded an allelic frequency of 37.3% for the type 2 diabetic patients and 30.5% for the glucose tolerant subjects ($p$ = 0.0007). The genotype relative risk for diabetes was estimated to 1.34 (95% confidence interval: 1.13 - 1.59) corresponding to a population attributable risk of 18%. Assuming an effect of the variant of 18% the combined study has a statistical power of approximately 90% for detecting a difference in allele frequency of the Gly482Ser polymorphism.

In the combined group of diabetic subjects, carriers of the Gly482Ser polymorphism did not differ significantly from wild type carriers in clinical or biochemical values including age of diabetes onset, BMI, waist circumference, treatment, degree and prevalence of micro- and macrovascular complications, $HbA_{1C}$ or fasting serum lipids (data not shown). Moreover, in the glucose tolerant subjects there was no evidence of a relation between the codon 482 variant and estimates of BMI, waist circumference, fasting serum triglycerides, serum total and HDL-cholesterol, plasma free fatty acids or plasma glucose, serum insulin and serum C-peptide during an OGTT (data not given).

**Table 1**

Primer sequences and PCR conditions for mutational analysis of *PGC-1*

| Primer | Sequence, 5'→3' | Location | $T_{anneal}$ | $MgCl_2$ |
|---|---|---|---|---|
| PC1F<br>PC1R | CTG GGG ACT GTA GTA AGA C<br>AGG GAA GCG TCA GTT GTG G | 5'UTR +<br>Exon 1 | 55°C | 1 mM |
| PC2F<br>PC2RNY | CCT GTG GTT AAT GGA AGC<br>GCC CAA GCC AAA CTC AAT G | Exon 2 | 50°C | 2 mM |
| PC3F<br>PC3R | CTG CCT CCC AGG GTC AAC<br>CAA CTC CAA TTC CTG CTA AAC | Exon 3 | 55°C | 1 mM |
| PC4F<br>PC4R | GAT GCA TAA CTT TAC TTG<br>CTG CTT CAA GCC AAA ATC | Exon 4 | 50°C | 2 mM |
| PC5F<br>PC5R | CTG ATA AGG TTC AGT TCA C<br>CCT CAC CAA CAG CTC GT | Exon 5 | 50°C | 2 mM |
| PC6F<br>PC6R | CCA ACT TGA CTG TTG TGG AG<br>ACA AAC TGA AAT GGA GTT GC | Exon 6 | 55°C | 2 mM |
| PC7F<br>PC7R | GGG TTC TAA TAC ATT TGG C<br>CAC ATA GAC AGT ACA TCT | Exon 7 | 50°C | 2 mM |
| PC8F<br>PC8R | GTT AAG TGG CAG TTG CAA ATG<br>GGG AGC TAA AGG AAA ATG AC | Exon 9 | 55°C | 2 mM |
| PC9F<br>PC9R | GGT GGT TGA CTT AGT GAT AAA G<br>CAC AGA AAA AGA AGA AAC CCT AC | Exon 10 | 55°C | 3 mM |
| PC10F<br>PC10R | CCA CTC CAG AAC TCT CTC C<br>CAA CTC CCA TCC CAG TAA TC | Exon 11 | 55°C | 1 mM |
| PC11F<br>PC11R | GGT TAC AGT CCC ATA TAC T<br>GAT TCC TCA TTC CAC GTA C | Exon 12 | 50°C | 3 mM |
| PC12F<br>PC12R | GCC ATC AGC AAA GTG TGT<br>TGA GGT ATT CGC CAT CCC | Exon 13 | 50°C | 2 mM |
| PC13F<br>PC14R | GAA ACA TGT GTC TTC GCA<br>CGC TTG GTC TTC CTT TCC TCG | Exon 8 | 55°C | 2 mM |
| PC15F<br>PC15R | CAA GTC CTC AGT CCT CAC<br>CTT GCC TCC AAA GTC TCT C | Exon 8 | 50°C | 2 mM |
| PC16F<br>PC16R | CAG ATT CAG ACC AGT G<br>CAT AGG TAG TTT GGA G | Exon 8 | 45°C | 1 mM |
| PC17F<br>PC17R | GGG ACA GTG ATT TCA GTA ATG<br>GGG GTC TTT GAG AAA ATA AGG | Exon 8 | 55°C | 1 mM |
| PC18F<br>PC18R | GTA GAG ATT CTG TGT CAC<br>CTT TTG TGT TAT TTA GGG | Exon 8 | 45°C | 2 mM |

All forward primers were extended with a 21M13 tail for sequencing (TGT AAA ACG ACG GCC AGT) and all reverse primers with an M13 tail (CAG GAA ACA GCT AGT ACC)

Table 2

| Genotype and allele frequencies of the examined variants in the *PGC-1* gene in type 2 diabetic patients and glucose tolerant subjects | | | |
|---|---|---|---|
| | Type 2 diabetic patients | Glucose tolerant subjects | *p* |
| | Initial association study | | |
| **Ser74Leu** | | | |
| Ser/Ser<br>Ser/Leu | 466 (99)<br>3 (1) | 197 (99)<br>1 (1) | |

Table 2 (continued)

| Genotype and allele frequencies of the examined variants in the *PGC-1* gene in type 2 diabetic patients and glucose tolerant subjects | | | |
|---|---|---|---|
| | Type 2 diabetic patients | Glucose tolerant subjects | *p* |
| | Initial association study | | |
| **Ser74Leu** | | | |
| Leu/Leu | 0 (0) | 0 (0) | |
| Allele frequency | 0.3 (0-0.7) | 0.3 (0-0.7) | 1.0 |
| **IVS2+52C→A** | | | |
| C/C | 178 (37) | 62 (30) | |
| C/A | 221 (46) | 102 (50) | |
| A/A | 79 (17) | 40 (20) | |
| Allele frequency | 39.6 (36.6-42.7) | 44.6 (39.8-49.4) | 0.09 |
| **Thr612Met** | | | |
| Thr/Thr | 443 (93) | 183 (90) | |
| Thr/Met | 31 (7) | 20 (10) | |
| Met/Met | 1 (0) | 0 (0) | |
| Allele frequency | 3.5 (2.3-4.6) | 4.9 (2.8-7.0) | 0.2 |
| **Gly482Ser** | | | |
| Gly/Gly | 186 (41) | 97 (49) | |
| Gly/Ser | 200 (44) | 80 (40) | |
| Ser/Ser | 68 (15) | 21 (11) | |
| Allele frequency | 37.0 (33.8-40.1) | 30.8 (26.2-35.3) | 0.032 |
| | Replication study | | |
| **Gly482Ser** | | | |
| Gly/Gly | 76 (38) | 146 (50) | |
| Gly/Ser | 97 (48) | 116 (40) | |
| Ser/Ser | 28 (14) | 31 (10) | |
| Allele frequency | 38.1 (33.3-42.8) | 30.4 (26.7-34.0) | 0.0135 |
| | Combined study | | |
| **Gly482Ser** | | | |
| Gly/Gly | 262 (40) | 243 (49) | |
| Gly/Ser | 297 (45) | 196 (40) | |
| Ser/Ser | 96 (15) | 52 (11) | |
| Allele frequency | 37.3 (34.7-39.9) | 30.5 (27.7-33.4) | 0.0007 |
| Data are number of subjects with each genotype (% of each group) and allele frequencies of minor allele in % (95% confidence interval). The *p*-values compare allele frequencies between type 2 diabetic patients and glucose tolerant subjects. | | | |

## Claims

1. An isolated polynucleotide molecule comprising a nucleotide sequence encoding PGC-1, said nucleotide sequence containing a mutation associated with type 2 diabetes of at least one nucleotide, or comprising a fragment of the nucleotide sequence including said mutation.

2. A polynucleotide according to claim 1 comprising a nucleotide sequence as shown in the Sequence Listing as SEQ ID NO: 1 containing a mutation associated with type 2 diabetes of at least one nucleotide or comprising a fragment of the nucleotide sequence shown in the Sequence Listing as SEQ ID NO: 1 including said mutation.

3. A polynucleotide according to claim 1 or claim 2, where said mutation gives rise to at least one amino acid substitution in PGC-1.

4. A polynucleotide according to any of claims 1 to 3, where said mutation gives rise to a substitution of Gly to Ser in the position corresponding to position 482 of the amino acid sequence shown in the Sequence Listing as SEQ ID NO: 2.

5. A polynucleotide according to any of claims 1 to 4, where said mutation corresponds to a mutation of G in position 1564 in SEQ ID NO: 1 to A.

6. A polynucleotide according to any of claims 1 to 5 wherein said polynucleotide is a DNA construct.

7. A recombinant vector, especially an expression vector, comprising a polynucleotide according to any of claims 1 to 6.

8. A cell line or a transgenic non-human mammal containing a polynucleotide according to any of claims 1 to 6 or a recombinant vector according to claim 7.

**9.** A cell line according to claim 8 wherein the cell line is a mammalian cell line.

**10.** A method of detecting the presence of a mutation in the gene encoding PGC-1, the method comprising obtaining a biological sample from a subject and analysing the sample for a mutation associated with type 2 diabetes of at least one nucleotide in the *PGC-1* sequence.

**11.** A method according to claim 10, wherein the sample is analysed for a mutation associated with type 2 diabetes of at least one nucleotide in the *PGC-1* sequence, which mutation gives rise to at least one amino acid substitution in PGC-1.

**12.** A method according to claim 10 or claim 11, wherein the sample is analysed for a mutation associated with type 2 diabetes of at least one nucleotide in the *PGC-1* sequence, which mutation gives rise to a substitution of Gly to Ser in the position corresponding to position 482 of the amino acid sequence shown in the Sequence Listing as SEQ ID NO: 2.

**13.** A method according to any of claims 10 to 12, wherein the sample is analysed for a mutation associated with type 2 diabetes of at least one nucleotide in the *PGC-1* sequence, which mutation corresponds to a mutation of G in position 1564 in SEQ ID NO: 1 to A.

**14.** A method according to any of claims 10 to 13, wherein a biological sample is obtained from a subject, DNA is isolated from the sample, the DNA is amplified and hybridised to a labelled polynucleotide comprising a nucleotide sequence encoding PGC-1, said nucleotide sequence containing a mutation associated with type 2 diabetes of at least one nucleotide, or comprising a fragment of the nucleotide sequence including said mutation, which mutation corresponds to the mutation the presence of which in the gene encoding PGC-1 is to be detected, and hybridisation of the labelled polynucleotide to the DNA is determined.

**15.** A method according to claim 14, wherein the labelled polynucleotide comprises a nucleotide sequence as shown in the Sequence Listing as SEQ ID NO: 1 containing a mutation associated with type 2 diabetes of at least one nucleotide or comprising a fragment of the nucleotide sequence shown in the Sequence Listing as SEQ ID NO: 1 including said mutation.

**16.** A method according to claim 14 or claim 15, wherein the labelled polynucleotide comprises a nucleotide sequence containing a mutation, which mutation gives rise to a substitution of Gly to Ser in the position corresponding to position 482 of the amino acid sequence shown in the Sequence Listing as SEQ ID NO: 2.

**17.** A method according to any of claims 14 to 16, wherein the labelled polynucleotide comprises a nucleotide sequence containing a mutation corresponding to a mutation of G in position 1564 in SEQ ID NO: 1 to A.

**18.** A method according to any of claims 14 to 17, wherein said labelled polynucleotide is a DNA construct.

**19.** A method according to any of claims 14 to 18, wherein the amplified DNA is hybridised to a second labelled polynucleotide comprising a DNA sequence corresponding to at least part of the wild-type gene encoding PGC-1, and hybridisation of said second labelled polynucleotide to the amplified DNA is determined.

**20.** A method according to claim 19, wherein the label substance with which the labelled polynucleotide carrying the mutation is labelled is different from the label substance with which the second labelled polynucleotide corresponding to at least part of the wild-type DNA is labelled.

**21.** A method according to any of claims 7 to 20 for determining predisposition to type 2 diabetes in a subject.

**22.** A diagnostic composition for determining predisposition to type 2 diabetes in a subject, the composition comprising a polynucleotide according to any of claims 1 to 6.

**23.** A diagnostic composition for detecting the presence of a mutation in the gene encoding PGC-1, the composition comprising a polynucleotide according to any of claims 1 to 6.

**24.** A test kit for detecting the presence of a mutation associated with type 2 diabetes in the gene encoding PGC-1, the kit comprising

(a) a first polynucleotide comprising a nucleotide sequence corresponding to at least part of the gene encoding PGC-1 and containing a mutation of at least one nucleotide, which mutation corresponds to the mutation the presence of which in the gene encoding PGC-1 is to be detected and optionally

(b) a second polynucleotide comprising a nucleotide sequence corresponding to at least part of the wild-type gene encoding PGC-1 and/or optionally

(c) a restriction endonuclease, which cleaves DNA at the site of the mutation.

**25.** A test kit according to claim 24, wherein the first polynucleotide is a polynucleotide according to any of claims 1 to 6.

**26.** A test kit according to claim 24 or claim 25, which further comprises means for amplifying DNA.

**27.** A test kit for detecting the presence of a mutation associated with type 2 diabetes in the gene encoding PGC-1, the kit comprising

(a) means for amplifying DNA, and

(b) a labelled polynucleotide comprising a nucleotide sequence corresponding to at least part of the gene encoding PGC-1 and containing a mutation of at least one nucleotide, which mutation corresponds to the mutation the presence of which in the gene encoding PGC-1 is to be detected.

**28.** A test kit according to claim 27, wherein the labelled polynucleotide comprises a polynucleotide according to any of claims 1 to 6.

**29.** A test kit according to claim 27 or claim 28, which comprises a second labelled polynucleotide comprising a nucleotide sequence corresponding to at least part of the wild-type gene encoding PGC-1.

**30.** A test kit according to claim 29, wherein the label substance with which the labelled polynucleotide carrying the mutation is labelled is different from the label substance with which the second labelled polynucleotide corresponding to at least part of the wild-type DNA is labelled.

**31.** A test kit according to claim 29 or claim 30, wherein the second labelled polynucleotide is a DNA construct.

**32.** A test kit according to any of claims 24 to 31 for determining predisposition to type 2 diabetes in a subject.

FIG. 1

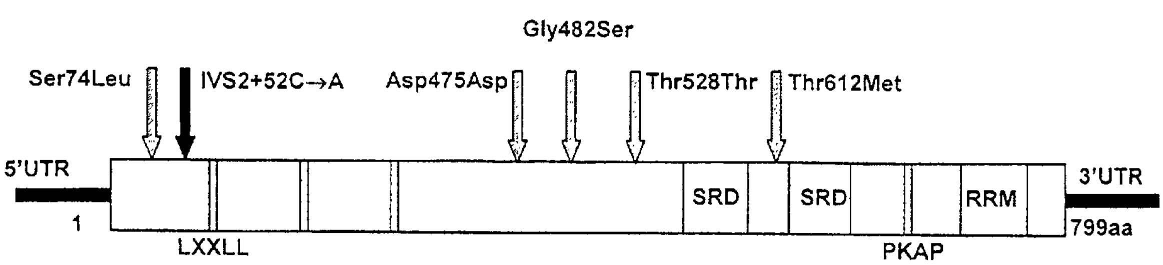
Gly482Ser
Ser74Leu
IVS2+52C→A
Asp475Asp
Thr528Thr
Thr612Met
5'UTR
1
SRD
SRD
RRM
3'UTR
799aa
LXXLL
PKAP

European Patent Office

# EUROPEAN SEARCH REPORT

Application Number
EP 01 61 0061

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98 54220 A (DANA FARBER CANCER INST INC) 3 December 1998 (1998-12-03) | 1-32 | C07K14/47<br>C12N15/12<br>C12N5/10<br>C12Q1/68<br>G01N33/53 |
| X | * see the whole document, in particular SEQ ID No:1 *<br>* figure 1A * | 1-6 | |
| X | * page 25, line 25 - page 33, line 12; examples IV,IX * | 7-9 | |
| X | * page 50, line 23 - page 53, line 9 * | 10-32 | |
| Y | * page 8, line 14 - page 9, line 2 *<br>--- | 10-32 | |
| X | WO 01 35096 A (ANDERSON CHRISTEN M ;CLEVENGER WILLIAM (US); MITOKOR (US); BECKER) 17 May 2001 (2001-05-17) | 1-9 | |
| X | * see SEQ ID No:6 * | 1-6 | |
| X | * page 68, line 19 - page 76, line 8 * | 7 | |
| X | * example 5 * | 8,9 | |
| X | -& DATABASE EM_PAT 'Online!<br>31 May 2001 (2001-05-31)<br>ANDERSON C M; BECKER D K; CLEVENGER W; GRAKO K A : "Diseases associated with altered mitochondrial function"<br>retrieved from EBI<br>Database accession no. AX146703<br>XP002181539<br>* the whole document *<br>--- | 1-6 | **TECHNICAL FIELDS SEARCHED (Int.Cl.7)**<br>C07K<br>C12N<br>C12Q |
| X | DATABASE EMBL 'Online!<br>LARROUY D; VIDAL H; ANDREELLI F; LAVILLE M; LANGIN D: "Cloning and mRNA tissue distribution of human PPARgamma coactivator-1."<br>retrieved from EBI<br>Database accession no. AF159714<br>XP002181540<br>* the whole document *<br>---<br>-/-- | 1-6 | |

The present search report has been drawn up for all claims

| Place of search | Date of completion of the search | Examiner |
|---|---|---|
| MUNICH | 31 October 2001 | Brenz Verca, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 01 61 0061

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y,D | MASATO ORITA ET AL: "DETECTION OF POLYMORPHISMS OF HUMAN DNA BY GEL ELECTROPHORESIS AS SINGLE-STRAND CONFORMATION POLYMORPHISMS" PROCEEDINGS OF THE NATIONAL ACADEMY OF SCIENCES OF USA, NATIONAL ACADEMY OF SCIENCE. WASHINGTON, US, vol. 86, no. 8, 1 April 1989 (1989-04-01), pages 2766-2770, XP000310584 ISSN: 0027-8424 * the whole document * | 10-32 | |
| A | ESTERBAUER HARALD ET AL: "Human peroxisome proliferator activated receptor gamma coactivator 1 (PPARGC1) gene: cDNA sequence, genomic organization, chromosomal localization, and tissue expression." GENOMICS, vol. 62, no. 1, 15 November 1999 (1999-11-15), pages 98-102, XP002181538 ISSN: 0888-7543 * see the whole document, in particular cited passage * * page 100, right-hand column, line 15 - line 27 * | 1-32 | TECHNICAL FIELDS SEARCHED (Int.Cl.7) |
| A,D | LANDEGREN U: "DETECTION OF MUTATIONS IN HUMAN DNA" GENETIC ANALYSIS TECHNIQUES AND APPLICATIONS, ELSEVIER SCIENCE PUBLISHING, NEW YORK, US, vol. 9, no. 1, 1 February 1992 (1992-02-01), pages 3-8, XP000276389 ISSN: 1050-3862 * the whole document * | 10-32 | |

The present search report has been drawn up for all claims

| Place of search | Date of completion of the search | Examiner |
|---|---|---|
| MUNICH | 31 October 2001 | Brenz Verca, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 61 0061

This annex lists the patent family members relating to the patent documents cited in the above-mentioned European search report.
The members are as contained in the European Patent Office EDP file on
The European Patent Office is in no way liable for these particulars which are merely given for the purpose of information.

31-10-2001

| Patent document cited in search report | | Publication date | Patent family member(s) | | Publication date |
|---|---|---|---|---|---|
| WO 9854220 | A | 03-12-1998 | EP | 1003777 A1 | 31-05-2000 |
| | | | US | 6166192 A | 26-12-2000 |
| | | | WO | 9854220 A1 | 03-12-1998 |
| WO 0135096 | A | 17-05-2001 | AU | 1763201 A | 06-06-2001 |
| | | | WO | 0135096 A2 | 17-05-2001 |

EPO FORM P0459

For more details about this annex : see Official Journal of the European Patent Office, No. 12/82

## SEQUENCE LISTING

<110> Novo Nordisk A/S

<120> Mutant DNA encoding peroxisome proliferator-activated receptor gamma coactivator-1

<130> 6311.000-EP

<160> 2

<170> PatentIn version 3.0

<210> 1

<211> 6303

<212> DNA

<213> Homo Sapiens

<220>

<221> polyA_signal

<222> (5164)..(5169)

<223> gene="PPARGC1"

<220>

<221> polyA_signal

<222> (6276)..(6281)

<223> gene="PPARGC1"

<400> 1

```
tagtaagaca ggtgccttca gttcactctc agtaaggggc tggttgcctg catgagtgtg      60

tgctctgtgt cactgtggat tggagttgaa aaagcttgac tggcgtcatt caggagctgg     120
```

atggcgtggg acatgtgcaa ccaggactct gagtctgtat ggagtgacat cgagtgtgct 180
gctctggttg gtgaagacca gcctctttgc ccagatcttc ctgaacttga tctttctgaa 240
ctagatgtga acgacttgga tacagacagc tttctgggtg gactcaagtg gtgcagtgac 300
caatcagaaa taatatccaa tcagtacaac aatgagcctt caaacatatt tgagaagata 360
gatgaagaga atgaggcaaa cttgctagca gtcctcacag agacactaga cagtctccct 420
gtggatgaag acggattgcc ctcatttgat gcgctgacag atggagacgt gaccactgac 480
aatgaggcta gtccttcctc catgcctgac ggcacccctc caccccagga ggcagaagag 540
ccgtctctac ttaagaagct cttactggca ccagccaaca ctcagctaag ttataatgaa 600
tgcagtggtc tcagtaccca gaaccatgca aatcacaatc acaggatcag aacaaaccct 660
gcaattgtta agactgagaa ttcatggagc aataaagcga agagtatttg tcaacagcaa 720
aagccacaaa gacgtccctg ctcggagctt ctcaaatatc tgaccacaaa cgatgacccct 780
cctcacacca aacccacaga gaacagaaac agcagcagag acaaatgcac ctccaaaaag 840
aagtcccaca cacagtcgca gtcacaacac ttacaagcca aaccaacaac tttatctctt 900
cctctgaccc cagagtcacc aaatgacccc aagggttccc catttgagaa caagactatt 960
gaacgcacct taagtgtgga actctctgga actgcaggcc taactccacc caccactcct 1020
cctcataaag ccaaccaaga taaccctttt agggcttctc caaagctgaa gtcctcttgc 1080
aagactgtgg tgccaccacc atcaaagaag cccaggtaca gtgagtcttc tggtacacaa 1140
ggcaataact ccaccaagaa agggccggag caatccgagt tgtatgcaca actcagcaag 1200
tcctcagtcc tcactggtgg acacgaggaa aggaagacca agcggcccag tctgcggctg 1260
tttggtgacc atgactattg ccagtcaatt aattccaaaa cggaaatact cattaatata 1320
tcacaggagc tccaagactc tagacaacta gaaaataaag atgtctcctc tgattggcag 1380
gggcagattt gttcttccac agattcagac cagtgctacc tgagagagac tttggaggca 1440
agcaagcagg tctctccttg cagcacaaga aaacagctcc aagaccagga aatccgagcc 1500
gagctgaaca agcacttcgg tcatcccagt caagctgttt ttgacgacga agcagacaag 1560
accggtgaac tgagggacag tgatttcagt aatgaacaat tctccaaact acctatgttt 1620
ataaattcag gactagccat ggatggcctg tttgatgaca gcgaagatga aagtgataaa 1680
ctgagctacc cttgggatgg cacacaatcc tattcattgt tcaatgtgtc tccttcttgt 1740
tcttctttta actctccatg tagagattct gtgtcaccac ccaaatcctt attttctcaa 1800
agaccccaaa ggatgcgctc tcgttcaagg tccttttctc gacacaggtc gtgttcccga 1860

tcaccatatt ccaggtcaag atcaaggtct ccaggcagta gatcctcttc aagatcctgc 1920
tattactatg agtcaagcca ctacagacac cgcacgcacc gaaattctcc cttgtatgtg 1980
agatcacgtt caagatcgcc ctacagccgt cggcccaggt atgacagcta cgaggaatat 2040
cagcacgaga ggctgaagag ggaagaatat cgcagagagt atgagaagcg agagtctgag 2100
agggccaagc aaagggagag gcagaggcag aaggcaattg aagagcgccg tgtgatttat 2160
gtcggtaaaa tcagacctga cacaacacgg acagaactga gggaccgttt tgaagttttt 2220
ggtgaaattg aggagtgcac agtaaatctg cgggatgatg gagacagcta tggtttcatt 2280
acctaccgtt atacctgtga tgcttttgct gctcttgaaa atggatacac tttgcgcagg 2340
tcaaacgaaa ctgactttga gctgtacttt tgtggacgca agcaattttt caagtctaac 2400
tatgcagacc tagattcaaa ctcagatgac tttgaccctg cttccaccaa gagcaagtat 2460
gactctctgg attttgatag tttactgaaa gaagctcaga gaagcttgcg caggtaacat 2520
gttccctagc tgaggatgac agagggatgg cgaatacctc atgggacagc gcgtccttcc 2580
ctaaagacta ttgcaagtca tacttaggaa tttctcctac tttacactct ctgtacaaaa 2640
acaaaacaaa acaacaacaa tacaacaaga acaacaacaa caataacaac aatggtttac 2700
atgaacacag ctgctgaaga ggcaagagac agaatgatat ccagtaagca catgtttatt 2760
catgggtgtc agctttgctt ttcctggagt ctcttggtga tggagtgtgc gtgtgtgcat 2820
gtatgtgtgt gtgtatgtat gtgtgtggtg tgtgtgcttg gtttagggga agtatgtgtg 2880
ggtacatgtg aggactgggg gcacctgacc agaatgcgca agggcaaacc atttcaaatg 2940
gcagcagttc catgaagaca cacttaaaac ctagaacttc aaaatgttcg tattctattc 3000
aaaaggaaaa atatatatat atatatatat atataaatta aaaaggaaag aaaactaaca 3060
accaaccaac caaccaacca accacaaacc accctaaaat gacagccgct gatgtctggg 3120
catcagcctt tgtactctgt ttttttaaga aagtgcagaa tcaacttgaa gcaagctttc 3180
tctcataacg taatgattat atgacaatcc tgaagaaacc acaggttcca tagaactaat 3240
atcctgtctc tctctctctc tctctctctc tctttttttt ttctttttcc ttttgccatg 3300
gaatctgggt gggagaggat actgcgggca ccagaatgct aaagtttcct aacattttga 3360
agtttctgta gttcatcctt aatcctgaca cccatgtaaa tgtccaaaat gttgatcttc 3420
cactgcaaat ttcaaaagcc ttgtcaatgg tcaagcgtgc agcttgttca gcggttcttt 3480
ctgaggagcg gacaccgggt tacattacta atgagagttg ggtagaactc tctgagatgt 3540
gttcagatag tgtaattgct acattctctg atgtagttaa gtatttacag atgttaaatg 3600

gagtattttt attttatgta tatactatac aacaatgttc ttttttgtta cagctatgca 3660
ctgtaaatgc agccttcttt tcaaaactgc taaatttttc ttaatcaaga atattcaaat 3720
gtaattatga ggtgaaacaa ttattgtaca ctaacatatt tagaagctga acttactgct 3780
tatatatatt tgattgtaaa aacaaaaaga cagtgtgtgt gtctgttgag tgcaacaaga 3840
gcaaaatgat gctttccgca catccatccc ttaggtgagc ttcaatctaa gcatcttgtc 3900
aagaaatatc ctagtcccct aaaggtatta accacttctg cgatattttt ccacattttc 3960
ttgtcgcttg tttttctttg aagttttata cactggattt gttaggggaa tgaaattttc 4020
tcatctaaaa tttttctaga agatatcatg attttatgta aagtctctca atgggtaacc 4080
attaagaaat gtttttattt tctctatcaa cagtagtttt gaaactagaa gtcaaaaatc 4140
tttttaaaat gctgttttgt tttaattttt gtgattttaa tttgatacaa aatgctgagg 4200
taataattat agtatgattt ttacaataat taatgtgtgt ctgaagacta tctttgaagc 4260
cagtatttct ttcccttggc agagtatgac gatggtattt atctgtattt tttacagtta 4320
tgcatcctgt ataaatactg atatttcatt cctttgttta ctaaagagac atatttatca 4380
gttgcagata gcctatttat tataaattat gagatgatga aaataataaa gccagtggaa 4440
attttctacc taggatgcat gacaattgtc aggttggagt gtaagtgctt catttgggaa 4500
attcagcttt tgcagaagca gtgtttctac ttgcactagc atggcctctg acgtgaccat 4560
ggtgttgttc ttgatgacat tgcttctgct aaatttaata aaaacttcag aaaaacctcc 4620
attttgatca tcaggatttc atctgagtgt ggagtccctg gaatggaatt cagtaacatt 4680
tggagtgtgt attcaagttt ctaaattgag attcgattac tgtttggctg acatgacttt 4740
tctggaagac atgatacacc tactactcaa ttgttctttt cctttctctc gccccaacacg 4800
atcttgtaag atggatttca cccccaggcc aatgcagcta attttgatag ctgcattcat 4860
ttatcaccag catattgtgt tctgagtgaa tccactgttt gtcctgtcgg atgcttgctt 4920
gatttttttgg cttcttattt ctaagtagat agaaagcaat aaaaatacta tgaaatgaaa 4980
gaacttgttc acaggttctg cgttacaaca gtaacacatc tttaatccgc ctaattcttg 5040
ttgttctgta ggttaaatgc aggtatttta actgtgtgaa cgccaaacta aagtttacag 5100
tctttctttc tgaattttga gtatcttctg ttgtagaata ataataaaaa gactattaag 5160
agcaataaat tattttaaga aatcgagatt tagtaaatcc tattatgtgt tcaaggacca 5220
catgtgttct ctattttgcc tttaaatttt tgtgaaccaa ttttaaatac attctccttt 5280
ttgccctgga ttgttgacat gagtggaata cttggtttct tttcttactt atcaaaagac 5340

agcactacag atatcatatt gaggattaat ttatcccccc tacccccagc ctgacaaata 5400
ttgttaccat gaagatagtt ttcctcaatg gacttcaaat tgcatctaga attagtggag 5460
cttttgtatc ttctgcagac actgtgggta gcccatcaaa atgtaagctg tgctcctctc 5520
atttttattt ttattttttt gggagagaat atttcaaatg aacacgtgca ccccatcatc 5580
actggaggca aatttcagca tagatctgta ggatttttag aagaccgtgg gccattgcct 5640
tcatgccgtg gtaagtacca catctacaat tttggtaacc gaactggtgc tttagtaatg 5700
tggatttttt tctttttaa aagagatgta gcaaaataat tcttccagtg caacaaaatc 5760
aatttttgc taaacgactc caagaacaac agttgggctg tcaacattca aagcagcaga 5820
gagggaactt tgcactattg gggtatgatg tttgggtcag ttgataaaag gaaacctttt 5880
catgccttta gatgtgagct tccagtaggt aatgattatg tgtcctttct tgatggctgt 5940
aatgagaact tcaatcactg tagtctaaga cctgatctat agatacctag aatagccatg 6000
tactataatg tgatgattct aaatttgtac ctatgtgaca gacattttca ataatgtgaa 6060
ctgctgattt gatggagcta ctttaagatt tgtaggtgaa agtgtaatac tgttggttga 6120
actatgctga agagggaaag tgagcgatta gttgagccct tgccgggcct tttttccacc 6180
tgccaattct acatgtattg ttgtggtttt attcattgta tgaaaattcc tgtgattttt 6240
tttaaatgtg cagtacacat cagcctcact gagctaataa agggaaacga atgtttcaaa 6300
tct 6303

<210> 2

<211> 798

<212> PRT

<213> Homo sapiens

<400> 2

Met Ala Trp Asp Met Cys Asn Gln Asp Ser Glu Ser Val Trp Ser Asp
1 5 10 15

Ile Glu Cys Ala Ala Leu Val Gly Glu Asp Gln Pro Leu Cys Pro Asp
20 25 30

Leu Pro Glu Leu Asp Leu Ser Glu Leu Asp Val Asn Asp Leu Asp Thr
35 40 45

```
Asp Ser Phe Leu Gly Gly Leu Lys Trp Cys Ser Asp Gln Ser Glu Ile
    50                  55                  60

Ile Ser Asn Gln Tyr Asn Asn Glu Pro Ser Asn Ile Phe Glu Lys Ile
65                  70                  75                  80

Asp Glu Glu Asn Glu Ala Asn Leu Leu Ala Val Leu Thr Glu Thr Leu
                85                  90                  95

Asp Ser Leu Pro Val Asp Glu Asp Gly Leu Pro Ser Phe Asp Ala Leu
            100                 105                 110

Thr Asp Gly Asp Val Thr Thr Asp Asn Glu Ala Ser Pro Ser Ser Met
        115                 120                 125

Pro Asp Gly Thr Pro Pro Pro Gln Glu Ala Glu Glu Pro Ser Leu Leu
    130                 135                 140

Lys Lys Leu Leu Leu Ala Pro Ala Asn Thr Gln Leu Ser Tyr Asn Glu
145                 150                 155                 160

Cys Ser Gly Leu Ser Thr Gln Asn His Ala Asn His Asn His Arg Ile
                165                 170                 175

Arg Thr Asn Pro Ala Ile Val Lys Thr Glu Asn Ser Trp Ser Asn Lys
            180                 185                 190

Ala Lys Ser Ile Cys Gln Gln Gln Lys Pro Gln Arg Arg Pro Cys Ser
        195                 200                 205

Glu Leu Leu Lys Tyr Leu Thr Thr Asn Asp Asp Pro Pro His Thr Lys
    210                 215                 220

Pro Thr Glu Asn Arg Asn Ser Ser Arg Asp Lys Cys Thr Ser Lys Lys
225                 230                 235                 240

Lys Ser His Thr Gln Ser Gln Ser Gln His Leu Gln Ala Lys Pro Thr
                245                 250                 255

Thr Leu Ser Leu Pro Leu Thr Pro Glu Ser Pro Asn Asp Pro Lys Gly
            260                 265                 270

Ser Pro Phe Glu Asn Lys Thr Ile Glu Arg Thr Leu Ser Val Glu Leu
        275                 280                 285

Ser Gly Thr Ala Gly Leu Thr Pro Pro Thr Thr Pro Pro His Lys Ala
    290                 295                 300

Asn Gln Asp Asn Pro Phe Arg Ala Ser Pro Lys Leu Lys Ser Ser Cys
305                 310                 315                 320

Lys Thr Val Val Pro Pro Pro Ser Lys Lys Pro Arg Tyr Ser Glu Ser
                325                 330                 335

Ser Gly Thr Gln Gly Asn Asn Ser Thr Lys Lys Gly Pro Glu Gln Ser
            340                 345                 350
```

```
Glu Leu Tyr Ala Gln Leu Ser Lys Ser Ser Val Leu Thr Gly Gly His
        355                 360                 365

Glu Glu Arg Lys Thr Lys Arg Pro Ser Leu Arg Leu Phe Gly Asp His
    370                 375                 380

Asp Tyr Cys Gln Ser Ile Asn Ser Lys Thr Glu Ile Leu Ile Asn Ile
385                 390                 395                 400

Ser Gln Glu Leu Gln Asp Ser Arg Gln Leu Glu Asn Lys Asp Val Ser
                405                 410                 415

Ser Asp Trp Gln Gly Gln Ile Cys Ser Ser Thr Asp Ser Asp Gln Cys
            420                 425                 430

Tyr Leu Arg Glu Thr Leu Glu Ala Ser Lys Gln Val Ser Pro Cys Ser
        435                 440                 445

Thr Arg Lys Gln Leu Gln Asp Gln Glu Ile Arg Ala Glu Leu Asn Lys
    450                 455                 460

His Phe Gly His Pro Ser Gln Ala Val Phe Asp Asp Glu Ala Asp Lys
465                 470                 475                 480

Thr Gly Glu Leu Arg Asp Ser Asp Phe Ser Asn Glu Gln Phe Ser Lys
                485                 490                 495

Leu Pro Met Phe Ile Asn Ser Gly Leu Ala Met Asp Gly Leu Phe Asp
            500                 505                 510

Asp Ser Glu Asp Glu Ser Asp Lys Leu Ser Tyr Pro Trp Asp Gly Thr
        515                 520                 525

Gln Ser Tyr Ser Leu Phe Asn Val Ser Pro Ser Cys Ser Ser Phe Asn
    530                 535                 540

Ser Pro Cys Arg Asp Ser Val Ser Pro Pro Lys Ser Leu Phe Ser Gln
545                 550                 555                 560

Arg Pro Gln Arg Met Arg Ser Arg Ser Arg Ser Phe Ser Arg His Arg
                565                 570                 575

Ser Cys Ser Arg Ser Pro Tyr Ser Arg Ser Arg Ser Arg Ser Pro Gly
            580                 585                 590

Ser Arg Ser Ser Ser Arg Ser Cys Tyr Tyr Tyr Glu Ser Ser His Tyr
        595                 600                 605

Arg His Arg Thr His Arg Asn Ser Pro Leu Tyr Val Arg Ser Arg Ser
    610                 615                 620

Arg Ser Pro Tyr Ser Arg Arg Pro Arg Tyr Asp Ser Tyr Glu Glu Tyr
625                 630                 635                 640

Gln His Glu Arg Leu Lys Arg Glu Glu Tyr Arg Arg Glu Tyr Glu Lys
                645                 650                 655
```

```
Arg Glu Ser Glu Arg Ala Lys Gln Arg Glu Arg Gln Arg Gln Lys Ala
            660                 665                 670

Ile Glu Glu Arg Arg Val Ile Tyr Val Gly Lys Ile Arg Pro Asp Thr
        675                 680                 685

Thr Arg Thr Glu Leu Arg Asp Arg Phe Glu Val Phe Gly Glu Ile Glu
    690                 695                 700

Glu Cys Thr Val Asn Leu Arg Asp Asp Gly Asp Ser Tyr Gly Phe Ile
705                 710                 715                 720

Thr Tyr Arg Tyr Thr Cys Asp Ala Phe Ala Ala Leu Glu Asn Gly Tyr
                725                 730                 735

Thr Leu Arg Arg Ser Asn Glu Thr Asp Phe Glu Leu Tyr Phe Cys Gly
            740                 745                 750

Arg Lys Gln Phe Phe Lys Ser Asn Tyr Ala Asp Leu Asp Ser Asn Ser
        755                 760                 765

Asp Asp Phe Asp Pro Ala Ser Thr Lys Ser Lys Tyr Asp Ser Leu Asp
    770                 775                 780

Phe Asp Ser Leu Leu Lys Glu Ala Gln Arg Ser Leu Arg Arg
785                 790                 795
```